# EUROPEAN PATENT APPLICATION

(11) **EP 3 705 122 A1**
(43) Date of publication of application: **09.09.2020**
(21) Application number: 18873632.6
(22) Date of filing: 01.11.2018
(51) Int. Cl.: A61K 31/44, A61K 9/06, A61K 9/10, A61K 9/14, A61K 47/32, A61K 47/34, A61K 47/36, A61K 47/42

(54) **SUSTAINED-RELEASE MEDICAL COMPOSITION**

(30) Priority: 02.11.2017 JP 2017213144
(71) Applicant: Ishihara Sangyo Kaisha, Ltd., Osaka-shi, Osaka 550-0002 (JP); Shizuoka Prefectural University Corporation, Shizuoka-shi, Shizuoka 422-8526 (JP)
(72) Inventor: ONOUE, Satomi, Shizuoka-shi Shizuoka 422-8526 (JP); SATO, Hideyuki, Shizuoka-shi Shizuoka 422-8526 (JP)
(74) Representative: Regimbeau
(86) International application number: PCT/JP2018/040634
(87) International publication number: WO 2019/088214

(57) **Abstract**

The present invention provides a composition for improving the sustained-release ability of N-(2-ethylsulfonylamino-5-trifluoromethyl-3-pyridyl)cyclohexan ecarboxamide or a salt thereof. More specifically, the present invention provides a composition for topical administration comprising N-(2-ethylsulfonylamino-5-trifluoromethyl-3-pyridyl)cyclohexan ecarboxamide or a salt thereof and a polycation polymer.

## Description

### CROSS-REFERENCE TO RELATED APPLICATIONS

This application is based upon and claims the benefit of priority from Japanese Patent Application No. 2017-213144, filed on November 2, 2017; the entire contents of which are incorporated herein by reference.

### TECHNICAL FIELD

### Field of the Invention

The present invention relates to a composition for topical administration comprising N-(2-ethylsulfonylamino-5-trifluoromethyl-3-pyridyl)cyclohexaneca rboxamide or a salt thereof, and more specifically relates to a composition for topical administration comprising N-(2-ethylsulfonylamino-5-trifluoromethyl-3-pyridyl)cyclohexaneca rboxamide or a salt thereof and a polycation polymer.

### BACKGROUND ART

It has been known that N-(2-ethylsulfonylamino-5-trifluoromethyl-3-pyridyl)cyclohexaneca rboxamide or a salt thereof has an inhibitory effect on phospholipase A2 and is useful as an active ingredient of an anti-inflammatory agent or an anti-pancreatitis agent (Patent Document 1).

It has also been known that a therapeutic agent or a prophylactic agent for gastrointestinal disease, liver disease, lung failure or shock containing N-(2-ethylsulfonylamino-5-trifluoromethyl-3-pyridyl)cyclohexaneca rboxamide or a salt thereof as an active ingredient is used (Patent Documents 2,3,4 and 5).

Furthermore, the above Patent Documents 1 to 5 also mention a drug formulation for oral administration, intravenous administration or subcutaneous administration comprising the above active ingredient. However, the above preparation cannot maintain an effective concentration of the above active ingredient, for example, in vivo for a long time, and may require plural administration in order to obtain a desired effect. Hence, it has been required to develop a technical means that can improve the sustained-release ability so that the desired effect can be exerted with less frequent dosing.

### Prior Art Document

### Patent Document

Patent Document 1
   JP H06-263735 A
Patent Document 2
   WO 2001/056568 A
Patent Document 3
   WO 2001/056569 A
Patent Document 4
   WO 2001/056570 A
Patent Document 5
   WO 2010/137484 A

### SUMMARY OF THE INVENTION

This time, the present inventors have found that administration of a specific composition comprising N-(2-ethylsulfonylamino-5-trifluoromethyl-3-pyridyl)cyclohexaneca rboxamide or a salt thereof to a living body improves the sustained-release ability of N-(2-ethylsulfonylamino-5-trifluoromethyl-3-pyridyl)cyclohexaneca rboxamide or a salt thereof.

Therefore, an object of the present invention is to provide a composition comprising N-(2-ethylsulfonylamino-5-trifluoromethyl-3-pyridyl)cyclohexaneca rboxamide or a salt thereof having enhanced sustained-release ability of N-(2-ethylsulfonylamino-5-trifluoromethyl-3-pyridyl)cyclohexaneca rboxamide or the salt thereof.

The present invention encompasses the following inventions:
(1) A composition for topical administration, comprising N-(2-ethylsulfonylamino-5-trifluoromethyl-3-pyridyl)cyclohe xanecarboxamide or a salt thereof, and
   a polycation polymer.
(2) The composition according to (1), wherein the polycation polymer is selected from the group consisting of a copolymer of an acrylic ester and a methacrylic ester having a positively charged nitrogen atom-containing group, polyamino acid, polyamine, polyamidoamine, polyimine, chitosan, poly N,N-dimethylaminoethyl methacrylate, polyvinylpyridine, polyimidazole, polyvinylamine, polyvinylformamide, protamine, polythiodiethylaminomethylethylene, poly-p-aminostyrene, polycation carbohydrate, polycation polymethacrylate, polycation polyacrylate, polycation polyoxetane, a derivative thereof and a salt thereof and a combination thereof.
(3) The composition according to (2), wherein the copolymer of an acrylic ester and a methacrylic ester having a positively charged nitrogen atom-containing group is an ethyl acrylate-methyl methacrylate-trimethylammoniumethyl methacrylate copolymer.
(4) The composition according to (2), wherein the polyamino acid is at least one selected from the group consisting of polylysine, polyarginine, polyhistidine and polyornithine.
(5) The composition according to any one of (1) to (4), wherein the polycation polymer is a biodegradable polymer.
(6) The composition according to any one of (1) to (5), wherein a form of the composition is a particle.
(7) The composition according to (6), wherein the particle is in a form of being suspended in a solvent.
(8) The composition according to any one of (1) to (5), wherein a form of the composition is a hydrogel.
(9) The composition according to (8), wherein the hydrogel further comprises a hydrophilic polymer selected from the group consisting of poly(alkyleneoxide), poly(vinylalcohol), alginic acid, hyaluronic acid, chondroitin sulfate, gelatin, dextran, polyethylene glycol, methylcellulose, hydroxymethyl cellulose, hydroxyethyl cellulose, polyhydroxybutyrate, poly(n-isopropylacrylamide), carrageenan, pectin, dextran sulfate and a combination thereof.
(10) The composition according to any one of (1) to (9), wherein the topical administration is subcutaneous administration, transrectal administration, intraperitoneal administration, intraarticular administration, intraocular administration, intratumoral administration, perivascular administration, intracranial administration, intramuscular administration, periocular administration, intrapalpebral administration, intraoral administration, intranasal administration, intravesical administration, intravaginal administration, intraurethral administration, intrarectal administration, adventitial administration or transnasal administration.
(11) The composition according to any one of (1) to (10), which is a sustained-release composition.
(12) The composition according to any one of (1) to (11), for treating or preventing a disease, a pathological condition or a symptom associated with an inflammatory cell.
(13) The composition according to any one of (1) to (12), for a non-human animal.
(14) A method for treating or preventing a disease, a pathological condition or a symptom associated with an inflammatory cell of a non-human animal, the method comprising topical administration of the composition according to any one of (1) to (13) to the non-human animal.
(15) A method for producing the composition according to any one of (1) to (14), the method comprising
   preparing a mixture comprising N-(2-ethylsulfonylamino-5-trifluoromethyl-3-pyridyl)cyclohexaneca rboxamide or a salt thereof and a polycation polymer.
(16) The production method according to (15), further comprising drying the mixture.
(17) The production method according to (16), wherein the drying is selected from the group consisting of spray drying, freeze drying and a combination thereof.
(18) The production method according to (15), wherein the mixture is a good solvent solution in which N-(2-ethylsulfonylamino-5-trifluoromethyl-3-pyridyl)cyclohexaneca rboxamide or a salt thereof and a polycation polymer are dissolved, the method comprising
   mixing the good solvent solution with a poor solvent.

According to the present invention, it is possible to effectively enhance the sustained-release ability of N-(2-ethylsulfonylamino-5-trifluoromethyl-3-pyridyl)cyclohexaneca rboxamide or a salt thereof.

### BRIEF DESCRIPTION OF THE DRAWINGS

FIG. 1 is a graph showing the results of an evaluation test of the elution of N-(2-ethylsulfonylamino-5-trifluoromethyl-3-pyridyl)cyclohexaneca rboxamide-monosodium salt-monohydrate (hereinafter also referred to as the compound 1) in a bulk powder of the compound 1 and compound 1-containing compositions (fine particles A to E). Data represent values of mean ± standard error (n = 2 to 3).
FIG. 2 is a graph showing concentration changes of the compound 1 in plasma of rats in the compound 1 bulk powder administration (subcutaneous administration, oral administration) group and a compound 1-containing composition (fine particle B). Data represent values of mean ± standard error (n = 3 to 6).
FIG. 3 is a graph showing the results of an evaluation test of the elution of the compound 1 in a compound 1 bulk powder and a compound 1-containing composition (fine particle F). Data represent values of mean ± standard error (n = 3).
FIG. 4 is a graph showing concentration changes of the compound 1 in plasma of rats in the compound 1 bulk powder administration group and a compound 1-containing composition (fine particle F). Data represent values of mean ± standard error (n = 6).
FIG. 5 is a graph showing concentration changes of the compound 1 in plasma of rats in the compound 1 bulk powder administration group and a compound 1-containing composition (hydrogel a). Data represent values of mean ± standard error (n = 4 to 6).
FIG. 6 is a graph showing concentration changes of the compound 1 in plasma of rats in the compound 1 bulk powder administration group and a compound 1-containing composition (hydrogel A). Data represent values of mean ± standard error (n = 4 to 6).

### DETAILED DESCRIPTION OF THE INVENTION

One of the characteristics of a composition of the present invention is to comprise a polycation polymer together with N-(2-ethylsulfonylamino-5-trifluoromethyl-3-pyridyl)cyclohexaneca rboxamide or a salt thereof.

### N-(2-Ethylsulfonylamino-5-trifluoromethyl-3-pyridyl)cyclohexaneca rboxamide or salt thereof

N-(2-Ethylsulfonylamino-5-trifluoromethyl-3-pyridyl)cyclohe xanecarboxamide in the present invention is represented by the structure formula of the following formula (1). Hereinafter, N-(2-ethylsulfonylamino-5-trifluoromethyl-3-pyridyl)cyclohexaneca rboxamide is sometimes abbreviated as a compound of formula (1).

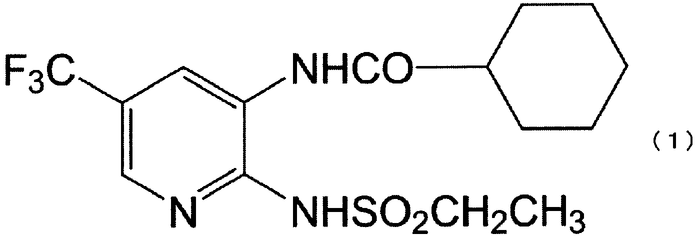

A salt of the compound of formula (1) may be a pharmaceutically acceptable salt, and examples thereof include alkali metal salts such as a potassium salt and a sodium salt; alkaline earth metal salts such as a calcium salt; organic amine salts such as a triethanolamine salt, and a tris(hydroxymethyl)aminomethane salt, and the like. Furthermore, the salt of the compound of formula (1) may be one having water of crystallization among these salts, namely a hydrate.

The compound of formula (1) or a salt thereof can be produced, for example, by the method mentioned in JP H06-263735 A.

A content of N-(2-ethylsulfonylamino-5-trifluoromethyl-3-pyridyl)cyclohexaneca rboxamide or a salt thereof in the composition of the present invention is not particularly limited as long as the effects of the present invention are not impaired, and examples thereof include 0.01 to 50% by mass, and it can be preferably 0.05 to 30% by mass, and more preferably 0.1 to 15% by mass or 0.1 to 20% by mass, based on the whole composition.

### Polycation polymer

The polycation polymer used in the present invention is not particularly limited, and includes ones that are used or that will be used in the future as drugs or foods.

The polycation polymer of the present invention is not particularly limited as long as the effects of the present invention are exerted if it is a positively charged polymer. Examples of a suitable polycation polymer include a polymer having a positively charged nitrogen atom-containing group such as an amino group, an ammonium group and an imino group (positively charged nitrogen atom-containing polycation polymer). Here, examples of the amino group include primary, secondary and tertiary amino groups, and examples of the ammonium group include primary, secondary, tertiary and quaternary ammonium groups. The above positively charged nitrogen atom-containing group encompasses not only a group containing a positively charged nitrogen atom but also a group containing a nitrogen atom that can be positively charged.

Specific examples of the above polycation polymer include a copolymer of an acrylic ester and a methacrylic ester having a positively charged nitrogen atom-containing group, polyamino acid, polyamine, polyamidoamine, polyimine, chitosan, poly N,N-dimethylaminoethyl methacrylate, polyvinylpyridine, polyimidazole, polyvinylamine, polyvinylformamide, protamine, polythiodiethylaminomethylethylene, poly-p-aminostyrene, polycation carbohydrate, polycation polymethacrylate, polycation polyacrylate, polycation polyoxetane, a derivative thereof and a salt thereof and a combination thereof and the like, and the above polycation polymer is preferably a copolymer of an acrylic ester and a methacrylic ester having a positively charged nitrogen atom-containing group, polyamino acid, polyamine, protamine and a salt thereof. Here, the copolymer of an acrylic ester and a methacrylic ester having a positively charged nitrogen atom-containing group is preferably an ethyl acrylate-methyl methacrylate-trimethylammoniumethyl methacrylate copolymer. Examples of the polyamino acid include polyamino acid having a positively charged nitrogen atom-containing group such as polylysine, polyarginine, polyhistidine and polyornithine, and the polyamino acid is preferably polylysine and polyarginine. Here, examples of the polylysine include α-polylysine and ε-polylysine, and ε-polylysine is preferred. Examples of the polyamine include spermine, spermidine, putrescine and the like. Examples of the above salt include preferably a pharmaceutically acceptable salt, and more preferably sulfate and hydrochloride. As the polycation polymer, a biodegradable polymer may be used in terms of safety and biocompatibility.

As the above polycation polymer, commercially available one may be used. Examples thereof include a copolymer of an acrylic ester and a methacrylic ester such as trade name (the same hereinafter) Eudragit (registered trademark) (manufactured by Evonik Industries AG); an ethyl acrylate-methyl methacrylate-trimethylammoniumethyl methacrylate copolymer such as Eudragit (registered trademark) RS including Eudragit (registered trademark) RS100, PO, 30D, 12,5 and the like, and Eudragit (registered trademark) RL including Eudragit (registered trademark) RL100, PO, 30D, 12,5 (manufactured by Evonik Industries AG) and the like, etc.

The above polymer may be used alone, or two or more polymers may be used in combination if necessary. When Eudragit (registered trademark) RS and Eudragit (registered trademark) RL are used, the release duration of the compound of formula (1) or a salt thereof can be controlled depending on their mixing ratio. Examples of the mixing ratio of Eudragit RS and Eudragit RL (Eudragit RS:Eudragit RL) include 100:0 to 0:100, and the mixing ratio is preferably 75:25 to 25:75, and more preferably 75:25 to 50:50 in terms of release control.

Examples of a weight average molecular weight of the polycation polymer of the present invention include 500 or more, and the weight average molecular weight can be preferably 500 to 200,000, and more preferably 2,000 to 50,000. The weight average molecular weight can be measured by size exclusion chromatography.

The compound of formula (1) or a salt thereof and the polycation polymer may form a complex (polyion complex), and in this case, the sustained-release ability is expected to be improved. From this point of view, the above polycation polymer may be one that can form a polyion complex with the compound of formula (1) or a salt thereof.

A content of the polycation polymer in the composition of the present invention is not particularly limited as long as the effects of the present invention are not impaired, and examples thereof include 0.01 to 99% by mass, and it can be preferably 0.05 to 95% by mass, and more preferably 0.1 to 90% by mass, based on the whole composition.

In the composition of the present invention, a mass ratio of the compound of formula (1) or a salt thereof to the polycation polymer (compound of formula (1) or salt thereof: polycation polymer) is not particularly limited as long as the effects of the present invention are not impaired, and, for example, it can be 1:0.01 to 1:100, preferably 1:0.2 to 1:70, and more preferably 1:1 to 1:10 or 1:0.5 to 1:10.

### Hydrophilic polymer

When a form of the composition of the present invention is a hydrogel, the composition may include a hydrophilic polymer. The hydrophilic polymer used is not particularly limited, and includes ones that are used or that will be used in the future as drugs or foods.

The hydrophilic polymer used for the hydrogel is preferably a high molecular weight substance that swells to become a gel when coming in contact with water (water swellable polymer) or a high molecular weight substance that becomes a gel at a specific temperature (thermosensitive polymer). Examples of the above hydrophilic polymer include poly(alkyleneoxide), poly(vinylalcohol), alginic acid, hyaluronic acid, chondroitin sulfate, gelatin, dextran, polyethylene glycol, hydroxymethyl cellulose, polyhydroxybutyrate, poly(n-isopropylacrylamide), carrageenan, pectin, dextran sulfate, poly(acrylic acid), hydroxypropylmethyl cellulose, hydroxypropyl cellulose, methylcellulose, carboxymethylcellulose sodium, hydroxyethyl cellulose, polyvinylpyrrolidone, a carboxyvinyl polymer and a combination thereof. As the above hydrophilic polymer, high molecular compounds having a clouding point may be used in order to form a gel at a specific temperature. These high molecular compounds can be prepared, for example, by a method of polymerizing a monomer having both a hydrophilic group such as an amide group and a carbonyl group and a hydrophobic group such as a linear or branched alkyl group or cycloalkyl group in the molecule, and by a method of introducing a hydrophilic group and a hydrophobic group into the high molecular compounds.

The above hydrophilic polymer may be used alone, or optional two or more hydrophilic polymers may be used in combination if necessary. A preferred hydrophilic polymer is poly(alkyleneoxide), hyaluronic acid and pectin, and a more preferred hydrophilic polymer is poly(ethyleneoxide).

As these hydrophilic polymers, commercially available ones may be used. Examples thereof include poly(ethyleneoxide) of trade name (the same hereinafter) : Poly(ethyleneoxide) [weight average molecular weight: 8,000,000, viscosity: 10,000 to 15,000 mPa·s (1% aqueous solution at 25°C)] (manufactured by Sigma Aldrich Co. LLC), Polyox WSR Coagulant [weight average molecular weight: 5,000,000, viscosity: 5,500 to 7,500 mPa·s (1% aqueous solution at 25°C)] (manufactured by The DOW Chemical Company), Polyox WSR-301 [mean molecular weight: 4,000,000, viscosity: 1,650 to 5,500 mPa·s (1% aqueous solution at 25°C)] (manufactured by The DOW Chemical Company), Polyox WSR-N-60K [weight average molecular weight: 2,000,000, viscosity: 2,000 to 4,000 mPa·s (2% aqueous solution at 25°C)] (manufactured by The DOW Chemical Company), Polyox WSR-N-12K [weight average molecular weight: 1,000,000, viscosity: 400 to 800 mPa·s (2% aqueous solution at 25°C)] (manufactured by The DOW Chemical Company), Polyox WSR-1105 [weight average molecular weight: 900,000, viscosity: 8,800 to 17,600 mPa·s (5% aqueous solution at 25°C)] (manufactured by The DOW Chemical Company), Polyox WSR-205 [weight average molecular weight: 600,000, viscosity: 4,500 to 8,800 mPa·s (5%aqueous solution at 25°C)] (manufactured by The DOW Chemical Company), Polyox WSR-N-750 [weight average molecular weight: 300,000, viscosity: 600 to 1,200 mPa·s (5% aqueous solution at 25°C)] (manufactured by The DOW Chemical Company), Polyox WSR-N-80 [weight average molecular weight: 200,000, viscosity: 55 to 90 mPa·s (5% aqueous solution at 25°C)] (manufactured by The DOW Chemical Company), Polyox WSR-N-10 [weight average molecular weight: 100,000, viscosity: 12 to 50 mPa·s (5% aqueous solution at 25°C)] (manufactured by The DOW Chemical Company) and the like.

Examples of a weight average molecular weight of the above hydrophilic polymer include 100,000 or more, and the weight average molecular weight can be preferably 100,000 to 10,000,000, and more preferably 500,000 to 9,000,000. Alternatively, examples of a viscosity of the hydrophilic polymer as the viscosity of 1% aqueous solution at 25°C include 1 mPa·s or more, and the viscosity is preferably 1,000 Pa·s, more preferably 2,000 to 50,000 mPa·s, and still more preferably 5,000 to 30,000 mPa·s.

In the hydrogel of the present invention, by adjusting the viscosity or weight average molecular weight of the hydrophilic polymer, it is possible to optionally control the control duration of the compound of formula (1) or a salt thereof from the composition.

According to a preferred embodiment of the hydrogel of the present invention, the polycation polymer used for the hydrogel is selected from the group consisting of a copolymer of an acrylic ester and a methacrylic ester having a positively charged nitrogen atom-containing group, polyamino acid, polyamine, protamine, a derivative thereof and a salt thereof and a combination thereof, and the hydrophilic polymer used for the hydrogel is selected from the group consisting of poly(alkyleneoxide), hyaluronic acid, pectin and a combination thereof.

According to another preferred embodiment of the hydrogel of the present invention, the polycation polymer used for the hydrogel is polyamine, and the hydrophilic polymer used for the hydrogel is poly(alkyleneoxide).

The composition of the present invention contains a pharmaceutically or orally acceptable additive as needed. The additive is not particularly limited, and examples thereof include aqueous vehicles such as purified water, solvents, bases, solubilizing agents, isotonizing agents, stabilizers, preservatives, antiseptics, surfactants, adjusters, chelating agents, pH adjusters, buffers, excipients, thickeners, coloring agents, aromatics, fragrances, antioxidants, dispersants, disintegrants, plasticizers, emulsifiers, solubilizers, reducing agents, sweetening agents, corrigents, binders and the like. The additive can be mixed insofar as the effects of the present invention are not impaired. Here, examples of the base include caprylic/capric triglyceride and a polylactic acid-polyglycolic acid copolymer (hereinafter also referred to as PLGA). As the caprylic/capric triglyceride, commercially available products can be used, and examples thereof include COCONARD MT (manufactured by Kao Corporation). As the PLGA, commercially available products can be used, and examples thereof include RESOMER RG503 (manufactured by Sigma Aldrich Co. LLC). As the surfactant, each of nonionic, anionic, cationic and amphoteric surfactants usually used in this technical field can be appropriately selected and used, and examples thereof include sorbitan monooleate, polyglyceryl polyricinoleate and the like. As the sorbitan monooleate, commercially available products can be used, and examples thereof include SPAN 80 (manufactured by Croda International PLC). Also, as the polyglyceryl polyricinoleate, commercially available products can be used, and examples thereof include NIKKOL Hexaglyn PR-15 (polyglyceryl-6 polyricinoleate) (manufactured by NIPPON SURFACTANT INDUSTRIES CO., LTD.).

The composition of the present invention may be in any form as long as the effects of the present invention are not impaired. Examples of the above composition include a liquid (including an oil and a slurry), a semisolid (including a paste and a gel) and a solid, and the above composition is preferably a particle and a hydrogel. Here, the particle may be in a form being suspended in a solvent, and examples of the solvent include water, physiological saline, a vegetable oil and propylene glycol. The hydrogel of the present invention may be a liquid (including an oil and a slurry) or a gel at the time of administration. However, although the hydrogel is a liquid or a solid (powder) at the time of administration, it may be a hydrogel in situ such as in vivo after administration, and the hydrogel of the present invention also encompasses the embodiment. The composition comprising the compound of formula (1) or a salt thereof of the present invention includes a combination of the compound of formula (1) or a salt thereof and the polycation polymer, but the dosage form is not particularly limited as long as the characteristics of the combination are maintained, and it can be provided as an injection, a suppository, an ointment, a cream, a gel, a patch, a drop, an eye drop, a nasal drop, an eye ointment, a cataplasm, a liniment, a lotion, a cream, a suspension, an emulsion, a syrup, an oral jelly, an implantable injection, a long-acting injection, a rectal semisolid, an enema agent and the like. The above dosage form is preferably a dosage form for topical administration, and examples thereof include an injection, a suppository and the like. Here, the injection includes a form of a kit comprising the composition of the present invention and a solvent in which the composition is suspended, and examples of the solvent include water, physiological saline, a vegetable oil and propylene glycol.

According to the present invention, topical administration of the above composition can effectively deliver the compound of formula (1) or a salt thereof into the body.

Here, topical administration of the present invention refers to an administration form in which the compound of formula (1) or a salt thereof is retained locally (at an administration site) to be absorbed in the body. A composition for topical administration of the present invention can be suitably used for not only a local action but also a systemic action.

Examples of the topical administration include parenteral administration, such as intramuscular administration, subcutaneous administration, intradermal administration, transmucosal administration such as transrectal administration, transdermal administration, intranasal administration, intraoral administration, intraperitoneal administration, intraarticular administration, intraocular administration, intratumoral administration, perivascular administration, intracranial administration, periocular administration, intrapalpebral administration, intravesical administration, intravaginal administration, intraurethral administration, intrarectal administration, adventitial administration, transnasal administration and the like. According to a preferred embodiment of the present invention, the composition of the present invention is provided as a composition for subcutaneous administration.

As shown in Examples mentioned later, the composition of the present invention can remarkably improve the sustained-release ability of the compound of formula (1) or a salt thereof. Therefore, according to a preferred embodiment of the present invention, the composition of the present invention is provided as a sustained-release composition of the compound of formula (1) or a salt thereof.

The composition of the present invention can be produced by mixing the compound of formula (1) or a salt thereof with the polycation polymer. The mixing method is not particularly limited as long as the composition of the present invention is obtained and the method does not impair the effects of the present invention, and examples thereof include kneading by a heat melting method, etc., a precipitation method such as an emulsion solvent diffusion method, etc., a bottom-up method, a mechanochemical method and the like. As an example, preparing a mixture including the compound of formula (1) or a salt thereof, the polycation polymer and, as needed, a solvent is exemplified.

When the composition of the present invention is obtained as a mixture including a solvent, the above production method may further include drying or cooling the above mixture. The above drying is not limited as long as the method can fully dry the solvent, and examples thereof include spray drying, freeze drying and a combination thereof. In terms of drying efficiency, powder recovery rate, economy and production scale up, spray drying is preferred.

The solvent used for preparation of the above mixture is not particularly limited, and includes ones that are used or that will be used in the future as drugs or foods. Specific examples of the above solvent include water, aliphatic halogenated hydrocarbons (e.g., dichloromethane, dichloroethane, chloroform, etc.), alcohols (e.g., methanol, ethanol, propanol, etc.), ketones (e.g., acetone, methyl ethyl ketone, etc.), ethers (e.g., diethyl ether, dibutyl ether, 1,4-dioxane, etc.), aliphatic hydrocarbons (e.g., n-hexane, cyclohexane, n-heptane, etc.), aromatic hydrocarbons (e.g., benzene, toluene, xylene, etc.), organic acids (e.g., acetic acid, propionic acid, etc.), esters (e.g., ethyl acetate, etc.), amides (e.g., dimethylformamide, dimethylacetamide, etc.) or a mixed solvent thereof, and water, ethanol, methanol, acetone, ethyl acetate or a mixed solvent thereof is preferred.

As another embodiment of the above production method, the above mixture is a good solvent solution in which the compound of formula (1) or a salt thereof and the polycation polymer are dissolved, and mixing the good solvent solution and a poor solvent may be further included. In other words, the production method of the composition of the present invention may be an emulsion solvent diffusion method.

An example of specific production methods will be shown below. First, the compound of formula (1) or a salt thereof is mixed with a good solvent, and the mixture (mixed solution) thus obtained is mixed with a polycation polymer solution to obtain a good solvent solution in which the compound of formula (1) or a salt thereof and the polycation polymer are dissolved. Thereafter, the above good solvent solution is mixed with a poor solvent by adding dropwise, etc., to obtain a suspension including the compound of formula (1) or a salt thereof and the polycation polymer. Thereafter, the above suspension is washed and frozen, followed by drying to obtain a composition including the compound of formula (1) or a salt thereof and the polycation polymer. The composition thus obtained is preferably a particle, and more preferably a fine particle such as a nanoparticle.

The above good solvent is not particularly limited as long as the solvent can dissolve the compound of formula (1) or a salt thereof. Examples thereof include water, ethanol and isopropyl alcohol, and the above good solvent is preferably water and ethanol.

A solvent used for the above polycation polymer solution is not particularly limited as long as the solvent can dissolve the polycation polymer. Examples thereof include acetone, butanol, ethyl acetate and dioxane, and the solvent is preferably acetone and butanol. The above polycation polymer solution may include a surfactant.

Therefore, the good solvent solution in which the compound of formula (1) or a salt thereof and the polycation polymer are dissolved may include a solvent in the polycation polymer solution, together with the good solvent.

The above poor solvent is not particularly limited as long as the solvent in which the compound of formula (1) or a salt thereof is difficult to dissolve. Examples thereof include hexane, diethyl ether, chloroform and tetrahydroxyfuran, and the above poor solvent is preferably hexane and diethyl ether. The above poor solvent may include a surfactant and a base.

As another embodiment of the above production method, when the composition is a hydrogel, for example, it is possible to prepare a mixture including the compound of formula (1) or a salt thereof, the polycation polymer, the hydrophilic polymer and, as needed, a solvent. Examples of the solvent include the same solvent as the solvent used for preparation of the mixture mentioned above, and the solvent is preferably water, ethanol, acetone and ethyl acetate.

Furthermore, the hydrogel thus obtained may be further dried to make a solid. The above drying is not limited as long as the method can fully dry the solvent, and examples thereof include spray drying, freeze drying and a combination thereof.

The composition for topical administration comprising the compound of formula (1) or a salt thereof of the present invention can be widely applied to a disease, a pathological condition or a symptom associated with an inflammatory cell (e.g., granulocytes (neutrophils, eosinophils, basophils), lymphocytes (e.g., T-lymphocytes, NK cells), monocytes, macrophages, plasma cells, mast cells, platelets) (e.g., pancreatitis, operative stress, disseminated intravascular coagulation (DIC), neoplastic disease, pyometra, heat stroke, immune-mediated hemolytic anemia (IMHA), sepsis, angiosarcoma, gastric volvulus, ischemia-reperfusion injury, purpura, liver failure, hepatitis, pneumonia, systemic inflammatory response syndrome (SIRS), trauma, osteoarthritis, cystitis, disk disease, atopy/allergy, dermatitis, immune-mediated disease, otitis, inflammatory bowel disease, chronic pain, colitis, chronic obstructive pulmonary disease (COPD), cholecystitis, cholangitis, etc.). Advantageously, the composition for topical administration of the present invention can exert treatment and preventive effects on pancreatitis, operative stress, disseminated intravascular coagulation (DIC) and the like. Therefore, according to another embodiment of the present invention, the composition of the present invention is provided as a composition for treatment or prevention of a disease, a pathological condition or a symptom associated with an inflammatory cell, preferably pancreatitis, operative stress or disseminated intravascular coagulation (DIC). The composition of the present invention can also be used as a drug and a quasi drug for humans or animals. The composition of the present invention may be appropriately used in combination with other drugs and quasi drugs that are regularly used in this technical field, as needed.

According to one embodiment, examples of a subject to which the composition of the present invention is applied include animals, and the subject is preferably non-human animals such as mammals, birds, reptiles, amphibians and fishes, and more preferably mice, rats, rabbits, dogs, cats, pigs, cattle and horses. The above animal may be livestock, pets, domestic animals, wild animals and racing animals. The above subject may be healthy individuals (healthy animals) or may be patients (patient animals).

According to another embodiment of the present invention, a method for treating or preventing a disease a pathological condition or a symptom associated with an inflammatory cell of a subject, preferably pancreatitis, operative stress or disseminated intravascular coagulation (DIC), comprising topical administration of the composition of the present invention comprising an effective amount of the compound of formula (1) or a salt thereof, is provided. According to further another embodiment of the present invention, the above-mentioned method for preventing a disease, a pathological condition or a symptom associated with an inflammatory cell of a subject is regarded as a non-therapeutic method excluding medical practice when the subject is a healthy individual. The method for treating or preventing a disease, a pathological condition or a symptom, etc., associated with an inflammatory cell of a subject of the present invention can be performed in accordance with the contents mentioned herein for the composition of the present invention.

According to another embodiment of the present invention, a method for improving the sustained-release ability of the compound of formula (1) or a salt thereof, comprising topical administration of the composition of the present invention comprising an effective amount of the compound of formula (1) or a salt thereof to a subject, is provided.

The effective amount of the compound of formula (1) or a salt thereof of the present invention and the frequency of administration of the composition of the present invention are not particularly limited, and are appropriately determined by a person skilled in the art according to the type and purity of the compound of formula (1) or a salt thereof, the dosage form of the composition, the duration of drug release, and the type, nature, sex, age, symptoms, etc., of the subject. For example, the effective amount of the compound of formula (1) or a salt thereof is 0.01 to 1,000 mg/body weight kg, and preferably 0.05 to 500 mg/body weight kg. Examples of the frequency of administration include once per 1 to several days, once per several weeks, once per month and the like. The duration of release of the compound of formula (1) or a salt thereof from the composition of the present invention is not particularly limited since it varies depending on the type of the compound of formula (1) or a salt thereof, the dosage form of the composition, the dose or the administration site, etc. The lower limit is, for example, 3 hours or more, preferably 6 hours or more, and more preferably 12 hours or more, and the upper limit is, for example, 1 year or less, preferably 4 months or less, and more preferably 2 months or less.

According to another embodiment of the present invention, use of a combination of the compound of formula (1) or a salt thereof and the polycation polymer in the production of the composition for topical administration is provided. According to another preferred embodiment of the present invention, the above composition is used for treatment or prevention of a disease, a pathological condition or a symptom associated with an inflammatory cell, preferably pancreatitis, operative stress or disseminated intravascular coagulation (DIC).

According to another embodiment of the present invention, use of a combination of the compound of formula (1) or a salt thereof and the polycation polymer for topical administration is provided. According to another preferred embodiment of the present invention, use of the above combination for treatment or prevention of a disease, a pathological condition or a symptom associated with an inflammatory cell, preferably pancreatitis, operative stress or disseminated intravascular coagulation (DIC) is provided.

According to another embodiment of the present invention, a combination product of the compound of formula (1) or a salt thereof and the polycation polymer for topical administration is provided. According to another preferred embodiment of the present invention, the above combination product for treatment or prevention of a disease, a pathological condition or a symptom associated with an inflammatory cell, preferably pancreatitis, operative stress or disseminated intravascular coagulation (DIC) is provided.

All of the above embodiments of use, combination and the combination product can be performed in accordance with the mentions on the composition and the method of the present invention.

### EXAMPLES

The present invention will be more specifically described by way of Test Examples and Reference Examples, but the technical scope of the present invention is not limited to these examples. Unless otherwise specified, all of percentages and ratios used in the present invention are by mass. Unless otherwise specified, units and measurement methods mentioned herein are in accordance with the Japanese Industrial Standards (JIS).

Substances used in Test Examples and Reference Examples are as follows:

### Compound 1:

### N-(2-ethylsulfonylamino-5-trifluoromethyl-3-pyridyl)cyclohexaneca rboxamide-monosodium salt-monohydrate

Eudragit (registered trademark) RSPO: manufactured by Evonik Industries AG (hereinafter referred to as Eudragit RS)
Eudragit (registered trademark) RLPO: manufactured by Evonik Industries AG (referred to as Eudragit RL)
SPAN 80: manufactured by Croda International PLC
NIKKOL Hexaglyn PR-15: manufactured by NIPPON SURFACTANT INDUSTRIES CO., LTD.
COCONARD MT: manufactured by Kao Corporation
Polyethylene oxide: weight average molecular weight: 8,000,000, manufactured by Sigma Aldrich Co. LLC
ε-Polylysine: weight average molecular weight:3,500, manufactured by OKUNO CHEMICAL INDUSTRIES CO.,LTD.
PLGA: RESOMER RG503, manufactured by Sigma Aldrich Co. LLC Polyvinyl alcohol: weight average molecular weight: 31,000 to 50,000, manufactured by Sigma Aldrich Co. LLC
Protamine sulfate: manufactured by Wako Pure Chemical Industries, Ltd.

Apparatuses used in Test Examples and Reference Examples are as follows:
Vortex mixer; manufactured by IKA
Homogenizer: manufactured by Microtec Co., Ltd.
Refrigerated centrifuge: manufactured by Hitachi Koki Co., Ltd.
Deep freezer: manufactured by Nihon Freezer Co., Ltd.
Freeze dryer: FD-1000, manufactured by TOKYO RIKAKIKI CO., LTD.
Shaker: INCUBATOR M-100, manufactured by TAITEC CORPORATION
Ultra-high performance liquid chromatography: Aquity UPLC, manufactured by Waters K.K.
Fine particle production device: Spray Dryer ADL311S, manufactured by Yamato Scientific Co., Ltd.

Quantitative determination of the concentration of the compound 1 in the following Test Examples and Reference Examples was performed using ultra-high performance liquid chromatography with a single quadrupole mass spectrometer as a detector.

The above ultra-high performance liquid chromatography and mass spectrometry were performed under the following conditions:
Column: Aquity UPLC BEC C18 Column (manufactured by Waters K.K.)
Column temperature: 40°C
Mobile phase: HPLC grade methanol (A), 0.1% formic acid (B)
Gradient condition: 0 to 0.5 minute, 60% A; 0.5 to 3.5 minutes, 60 to 80% A
Flow rate: 0.25 mL/min
Injection volume: 5 µL
Sample temperature: 15°C
Retention time: 2.6 minutes
Ion mode: ES⁻
m/z: 378.1
Cone voltage: 60 V
Capillary voltage: 4.00 kV
Cone gas: 50 L/h
Source temperature: 120°C
Desolvation temperature: 400°C

### Reference Example 1: Investigation of Compound 1-Containing Composition (Fine Particle a)

### Reference Example 1-1: Preparation of Compound 1-Containing Composition (Fine Particle a)

A compound 1 (8 mg) and purified water (0.8 mL) were mixed using a vortex mixer to obtain liquid a. PLGA (40 mg), SPAN 80 (40 mg) and acetone (1.2 mL) were mixed using a vortex mixer to obtain liquid b. Liquid a and liquid b were mixed using a vortex mixer to obtain liquid c. NIKKOL Hexaglyn PR-15 (240 mg), COCONARD MT (12 mL) and hexane (8 mL) were mixed using a vortex mixer to obtain liquid d. Polyvinyl alcohol (100 mg) and purified water (10 mL) were stirred using a magnetic stirrer to obtain liquid e. Liquid c was added dropwise to liquid d, followed by stirring using a homogenizer at 15,000 rpm for 5 minutes to obtain a suspension. The suspension thus obtained was centrifuged using a refrigerated centrifuge at 4°C and 20,000 rpm for 10 minutes, and the supernatant was discarded to obtain a precipitate. To the precipitate thus obtained, hexane (10 mL) was added, followed by stirring using a vortex mixer for 5 minutes to obtain a suspension. The suspension was centrifuged using a refrigerated centrifuge at 4°C and 20,000 rpm for 10 minutes, and the supernatant was discarded to obtain a precipitate. To the precipitate thus obtained, liquid e (10 mL) was added, followed by stirring using a vortex mixer for 5 minutes to obtain a suspension. The suspension was centrifuged using a refrigerated centrifuge at 4°C and 20,000 rpm for 10 minutes, and the supernatant was discarded to obtain a precipitate. To the precipitate thus obtained, purified water (10 mL) was added, followed by stirring using a vortex mixer to obtain a suspension. The suspension was frozen using a deep freezer at -80°C overnight. After freezing, the frozen suspension was freeze-dried using a freeze dryer over two nights to obtain fine particle a.

### Reference Example 1-2: Elution Property of Compound 1-Containing Composition (Fine Particle a)

An evaluation test of the elution of the fine particle a prepared in Reference Example 1-1 was performed in the same manner as in Test Example 1 mentioned later. Specifically, the fine particle a (10 mg) was added to phosphate-buffered saline (pH 7.4, 30 mL), and using a shaker, the mixture was horizontally shaken at 37°C for 24 hours at a shaking speed of 60 strokes/min and with a shaking width of 2.0 cm/stroke to evaluate "elution". Quantitative determination of the concentration of the compound 1 was performed using ultra-high performance liquid chromatography with a single quadrupole mass spectrometer as a detector to calculate an elution rate. The test results of the fine particle a are shown in Table 1 below together with the test results of the compound 1 bulk powder obtained in Test Example 1 mentioned later.

**[Table 1]**

| | | Elution rate (%) | |
|---|---|---|---|
| | | Compound 1 bulk powder | Fine particle a |
| Time (h) | 0 | 0 | 0 |
| | 0.5 | 83.73 ± 4.31 | > 99 |
| | 1 | 93.59 ± 1.63 | > 99 |
| | 3 | 94.77 ± 2.26 | > 99 |
| | 6 | 92.00 ± 2.45 | > 99 |
| | 12 | 91.63 ± 1.84 | > 99 |
| | 24 | 97.10 ± 1.96 | > 99 |

In Table 1, values of the elution amount (n = 2 to 3) were expressed as mean ± standard error.

In Reference Example 1, achievement of the sustained release of the compound 1 was attempted by an emulsion solvent diffusion method using PLGA as a base, which has been used as a conventional method for preparing a sustained-release fine particle. However, the sustained-release ability could not be confirmed from the results of the elution test of the fine particle prepared. The elution rate of the fine particle a was more than 99%, and it was also revealed that this method could not control the amount of enclosed drug during particle preparation. Therefore, the sustained release of the compound 1-containing composition obtained by the emulsion solvent diffusion method using PLGA as a base could not be achieved, and it was confirmed for the first time that a method that had been conventionally used between persons skilled in the art could not be applied.

### Test Example 1: Investigation of Compound 1-Containing Compositions (Fine Particles A to E)

### Test Example 1-1: Preparation of Compound 1-Containing Compositions (Fine Particles A to E)

A compound 1 (8 mg) and purified water (0.8 mL) were mixed using a vortex mixer to obtain liquid A. A mixture of Eudragit RS and Eudragit RL (40 mg, Eudragit RS:RL = 100:0, 75:25, 50:50, 25:75 and 0:100), SPAN 80 (40 mg) and acetone (1.2 mL) were mixed using a vortex mixer to obtain liquid B. Liquid A and liquid B were mixed using a vortex mixer to obtain liquid C. NIKKOL Hexaglyn PR-15 (240 mg), COCONARD MT (12 mL) and hexane (8 mL) were mixed using a vortex mixer to obtain liquid D. Liquid C was added dropwise to liquid D, followed by stirring using a homogenizer at 15,000 rpm for 5 minutes to obtain a suspension. The suspension thus obtained was centrifuged using a refrigerated centrifuge at 4°C and 20,000 rpm for 10 minutes, and the supernatant was discarded to obtain a precipitate. To the precipitate thus obtained, hexane (10 mL) was added, followed by stirring using a vortex mixer for 5 minutes to obtain a suspension. The suspension was centrifuged using a refrigerated centrifuge at 4°C and 20,000 rpm for 10 minutes, and the supernatant was discarded to obtain a precipitate. To the precipitate thus obtained, 10 mL of purified water was added, followed by stirring using a vortex mixer to obtain a suspension. The suspension was frozen using a deep freezer at -80°C. After freezing, the frozen suspension was freeze-dried using a freeze dryer to obtain fine particles. Among the fine particles thus obtained, a particle having a ratio of Eudragit RS to RL (Eudragit RS:Eudragit RL) of 100:0 was regarded as fine particle A, a particle having a ratio of 75:25 was regarded as fine particle B, a particle having a ratio of 50:50 was regarded as fine particle C, a particle having a ratio of 25:75 was regarded as fine particle D, and a particle having a ratio of 0:100 was regarded as fine particle E.

### Test Example 1-2: Elution Property of Compound 1-Containing Compositions (Fine Particles A to E)

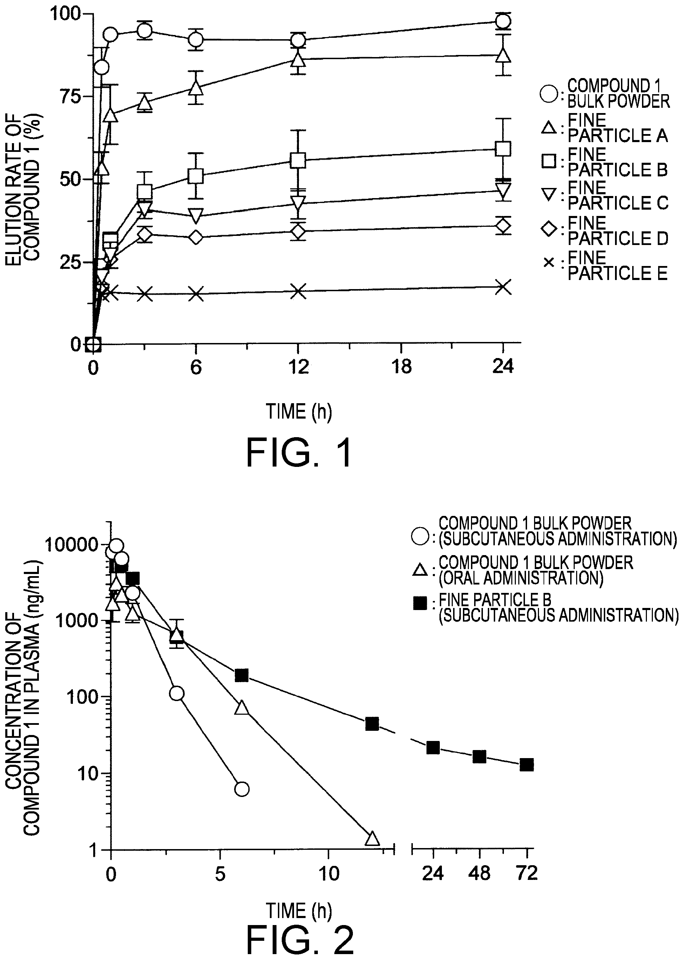

Using the fine particles A, B, C, D and E prepared in Test Example 1-1 and the compound 1 bulk powder, an evaluation test of each elution was performed. Each of the fine particles A, B, C, D and E and the compound 1 bulk powder (10 mg) was added to phosphate-buffered saline (pH 7.4, 30 mL), and using a shaker, the mixtures were horizontally shaken at 37°C for 24 hours at a shaking speed of 60 strokes/min and with a shaking width of 2.0 cm/stroke to evaluate "elution". Quantitative determination of the concentration of the compound 1 was performed using ultra-high performance liquid chromatography with a single quadrupole mass spectrometer as a detector to calculate an elution rate. The results are shown in Table 2 below and FIG. 1.

**[Table 2]**

| | | Elution rate (%) | | |
|---|---|---|---|---|
| | | Compound 1 bulk powder | Fine particle A | Fine particle B |
| Time (h) | 0 | 0 | 0 | 0 |
| | 0.5 | 83.73 ± 4.31 | 53.43 ± 3.33 | 23.95 ± 1.72 |
| | 1 | 93.59 ± 1.63 | 69.48 ± 6.38 | 31.31 ± 2.19 |
| | 3 | 94.77 ± 2.26 | 73.08 ± 2.04 | 46.10 ± 4.90 |
| | 6 | 92.00 ± 2.45 | 77.48 ± 3.56 | 50.77 ± 5.60 |
| | 12 | 91.63 ± 1.84 | 85.87 ± 3.21 | 55.31 ± 7.29 |
| | 24 | 97.10 ± 1.96 | 86.88 ± 4.38 | 58.59 ± 7.41 |
| | | | | |

| | | Elution rate (%) | | |
|---|---|---|---|---|
| | | Fine particle C | Fine particle D | Fine particle E |
| Time (h) | 0 | 0 | 0 | 0 |
| | 0.5 | 20.53 ± 1.88 | 17.84 ± 0.90 | 15.13 ± 1.35 |
| | 1 | 27.08 ± 3.18 | 25.95 ± 0.32 | 15.79 ± 1.07 |
| | 3 | 40.75 ± 2.09 | 33.37 ± 1.97 | 15.35 ± 0.47 |
| | 6 | 38.68 ± 0.99 | 32.38 ± 0.34 | 15.28 ± 0.37 |
| | 12 | 42.26 ± 3.63 | 34.01 ± 2.23 | 15.92 ± 0.30 |
| | 24 | 45.89 ± 2.51 | 35.51 ± 2.16 | 17.02 ± 0.09 |

In Table 2, values of the elution rate (n = 2 to 3) were expressed as mean ± standard error.

From the results of Table 2 and FIG. 1, each fine particle of Test Example 1 showed the property of a sustained-release particle.

By enclosing a water-soluble compound such as a compound 1 into a polycation polymer by the emulsion solvent diffusion method, it was possible to control the release velocity of the compound 1. Also, by changing the compositions of Eudragit RS and Eudragit RL, it was possible to control the release velocity. It is estimated that this is because, in the fine particle F prepared, the compound 1 is uniformly dispersed in Eudragit RS and Eudragit RL, and when water enters the particle, the compound 1 is gradually released from the surface of the fine particle.

### Test Example 1-3: Pharmacokinetic Evaluation of Compound 1-Containing Composition (Fine Particle B)

To evaluate the sustained-release ability of fine particle B, the concentration of the compound 1 in blood was measured over time after subcutaneous administration of the fine particle B or the compound 1 bulk powder to rats or after oral administration of the compound 1 bulk powder to rats. The fine particle B was suspended in physiological saline and the compound 1 bulk powder was dissolved in physiological saline, and then 5 mg/kg each as an amount of the compound 1 was singly administered to SD male rats. After administration, blood was collected over time from the tail vein, and the blood was transferred into a micro test tube treated with heparin, and immediately cooled in ice. After cooling in ice, the blood was immediately centrifuged at 4°C and 10,000 g for 10 minutes. The concentration of the compound 1 in plasma thus obtained was quantitatively determined using ultra-high performance liquid chromatography with a single quadrupole mass spectrometer as a detector.

The results are shown in FIG. 2. The following Table 3 shows the pharmacokinetic parameters calculated from the results of blood drug concentration measurement. Cmax, Tₘₐₓ, T_{1/2}, AUC_{0-∞} and BA represent maximum blood concentration (concentration at the peak of blood concentration curve), time to reach maximum blood concentration (time to reach the peak of blood concentration curve), half-life of the concentration of drug in blood, area under curve from initiation of administration to drug elimination and bioavailability, respectively.

**[Table 3]**

| | Cₘₐₓ (µg/mL) | Tₘₐₓ (h) | T_{1/2} (h) | AUC_{0-∞} (µg·h/mL) | BA (%) |
|---|---|---|---|---|---|
| Compound 1 bulk powder Subcutaneous administration | 9.6 ± 0.7 | 0.25 ± 0.0 | 0.36 ± 0.0 | 8.55 ± 0.6 | 67 |
| Compound 1 bulk powder Oral administration | 3.1 ± 0.5 | 0.25 ± 0.0 | 1.14 ± 0.2 | 5.16 ± 0.2 | 41 |
| Fine particle B Subcutaneous administration | 5.6 ± 0.3 | 0.42 ± 0.0 | 1.12 ± 0.0 | 11.3 ± 0.5 | 89 |

In Table 3, each pharmacokinetic parameter was expressed as mean ± standard error (n = 3 to 6).

As shown in the above Table 3 and FIG. 2, the results of evaluation of disposition at the time of oral administration and subcutaneous administration of the compound 1 bulk powder showed that the bioavailability at the time of subcutaneous administration was superior to that at the time of oral administration. The fine particle B prepared in Test Example 1 had decreased absorption and elimination velocity of the compound 1, compared with the compound 1 bulk powder that was subcutaneously administered. Furthermore, the fine particle B showed BA which was about 2-fold higher than that of the compound 1 bulk powder which was orally administered at the same dose. Here, the Cₘₐₓ of the fine particle B was decreased by 41.7% compared with that of the compound 1 bulk powder which was subcutaneously administered. The T_{1/2} of the fine particle B was prolonged by 0.76 hour compared with the T_{1/2} of the compound 1 bulk powder which was subcutaneously administered, and the fine particle B of Test Example 1 showed increased retention in blood of the compound 1. This is estimated to be due to the fact that the fine particle B prepared in Test Example 1 has a sustained-release drug elution property.

### Test Example 2: Investigation of Compound 1-Containing Composition (Fine Particle F)

### Test Example 2-1: Preparation of Compound 1-Containing Composition (Fine Particle F)

A compound 1 (600 mg), Eudragit RS (4050 mg) and Eudragit RL (1350 mg) (Eudragit RS:RL = 75:25), purified water (12 mL) and ethanol (28 mL) were mixed using a vortex mixer to obtain liquid E. The liquid E thus obtained was discharged to form droplets using a fine particle production device by a spray drying method, and dried to obtain fine particle F. The conditions are as follows:

### - Fine particle preparation conditions -

Inlet temperature: 130°C
Outlet temperature: 77°C or more and 82°C or less
Mean circulating air volume: 0.47 m³/min
Air pressure: 0.1 MPa
Nozzle diameter: 0.4 mm
Feed rate: 3.01 g/min
Trap temperature: -2°C

### Test Example 2-2: Elution Property of Compound 1-Containing Composition (Fine Particle F)

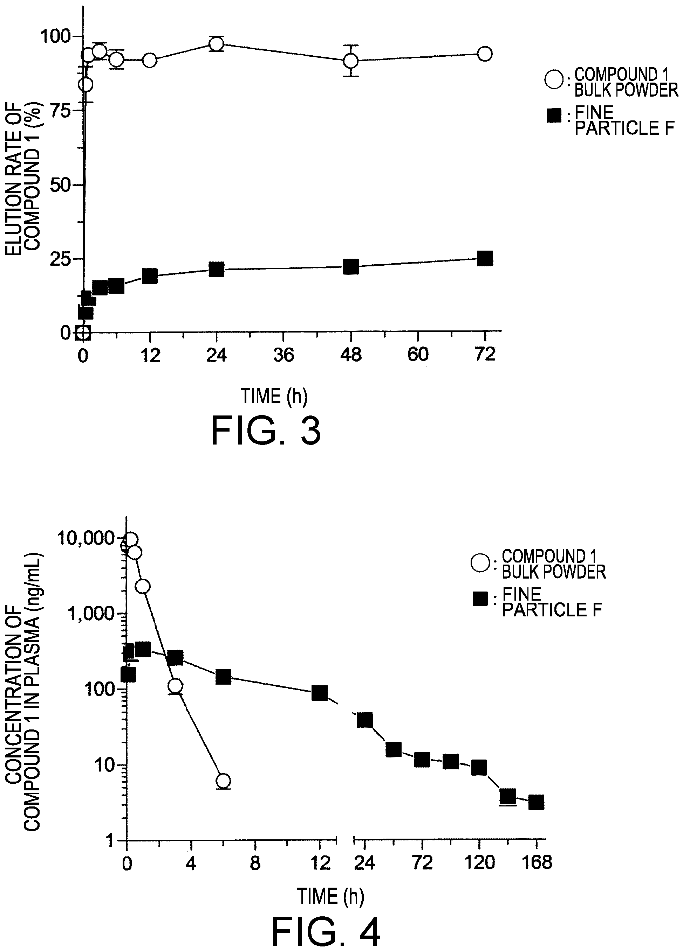

Using the fine particle F prepared in Test Example 2-1 and the compound 1 bulk powder, an evaluation test of each elution was performed in the same manner as in Test Example 1. Each of the fine particle F and the compound 1 bulk powder (10 mg) was added to phosphate-buffered saline (pH 7.4, 30 mL), and using a shaker, the mixtures were horizontally shaken at 37°C for 72 hours at a shaking speed of 60 strokes/min and with a shaking width of 2.0 cm/stroke to evaluate "elution". The results are shown in Table 4 below and FIG. 3.

**[Table 4]**

| | | Elution rate (%) | |
|---|---|---|---|
| | | Compound 1 bulk powder | Fine particle F |
| Time (h) | 0 | 0 | 0 |
| | 0.5 | 83.73 ± 4.31 | 7.09 ± 0.37 |
| | 1 | 93.59 ± 1.63 | 11.66 ± 1.54 |
| | 3 | 94.77 ± 2.26 | 15.15 ± 0.80 |
| | 6 | 92.00 ± 2.45 | 15.85 ± 0.53 |
| | 12 | 91.63 ± 1.84 | 19.06 ± 0.46 |
| | 24 | 97.10 ± 1.96 | 21.20 ± 0.44 |
| | 48 | 91.10 ± 3.80 | 21.95 ± 0.66 |
| | 72 | 93.29 ± 1.36 | 24.58 ± 1.49 |

In Table 4, values of the elution rate (n = 3) were expressed as mean ± standard error.

From the results of the above Table 4 and FIG. 3, the fine particle F of Test Example 2 showed the property of a sustained-release particle.

By enclosing a water-soluble compound such as a compound 1 into a polycation polymer by the spray drying method, it was possible to control the release velocity of the compound 1. It is estimated that this is because, in the fine particle F prepared, the compound 1 is uniformly dispersed in Eudragit RS and Eudragit RL, and when water enters the particle, the compound 1 is gradually released from the surface of the fine particle.

### Test Example 2-3: Pharmacokinetic Evaluation of Compound 1-Containing Composition (Fine Particle F)

To evaluate the sustained-release ability of fine particle F, the concentration of the compound 1 in blood was measured over time after subcutaneous administration of the fine particle F prepared in Test Example 2-1 or the compound 1 bulk powder to rats. The method was same as in Test Example 1-3. Specifically, the fine particle F was suspended in physiological saline and the compound 1 bulk powder was dissolved in physiological saline, and then 5 mg/kg each as an amount of the compound 1 was singly subcutaneously administered to SD male rats. After subcutaneous administration, blood was collected over time from the tail vein, and the blood was transferred into a micro test tube treated with heparin, and immediately cooled in ice. After cooling in ice, the blood was immediately centrifuged at 4°C and 10,000 g for 10 minutes. The concentration of the compound 1 in plasma thus obtained was quantitatively determined using ultra-high performance liquid chromatography with a single quadrupole mass spectrometer as a detector. The results are shown in FIG. 4. The following Table 5 shows the pharmacokinetic parameters calculated from the results of blood drug concentration measurement.

**[Table 5]**

| | Cₘₐₓ (µg/mL) | Tₘₐₓ (h) | T_{1/2} (h) |
|---|---|---|---|
| Compound 1 bulk powder | 9.6 ± 0.7 | 0.25 ± 0.0 | 0.36 ± 0.0 |
| Fine particle F | 0.36 ± 0.0 | 1.04 ± 0.4 | 5.55 ± 0.4 |

In Table 5, each pharmacokinetic parameter was expressed as mean ± standard error (n = 6).

From the results of the above Table 5 and FIG. 4, the fine particle F prepared in Test Example 2 had decreased absorption and elimination velocity of the compound 1, compared with the compound 1 bulk powder. Here, the Cₘₐₓ of the fine particle F was 27-fold lower than that of the compound 1 bulk powder. The T_{1/2} of the fine particle F was prolonged by 5.19 hour compared with that of the compound 1 bulk powder, and the fine particle F of Test Example 2 showed increased retention in blood of the compound 1. At 24 hours after administration, the concentration of the compound 1 bulk powder in plasma was below the detection limit, while the concentration of the fine particle F in plasma was 43 ng/mL. This is estimated to be due to the fact that the fine particle F prepared in Test Example 2 has a sustained-release drug elution property.

### Reference Example 2: Preparation of Compound 1-Containing Composition (Fine Particle b)

A compound 1 (100 mg), PLGA (500 mg) and a mixed solution of acetone and methanol (acetone: methanol (volume ratio) = 2:1, 50 mL) were mixed using a vortex mixer to obtain liquid f. The liquid f thus obtained was discharged to form droplets using a fine particle production device by a spray drying method, and dried to obtain fine particle b (yield: 35%). The conditions are as follows:

### - Fine particle preparation conditions -

Inlet temperature: 60°C
Outlet temperature: 50°C or less
Mean circulating air volume: 0.51 m³/min
Air pressure: 0.3 MPa
Nozzle diameter:0.4 mm
Feed rate: 5 mL/min
Trap temperature: -2°C

### Test Example 3: Preparation of Compound 1-Containing Composition (Fine Particle G)

A compound 1 (100 mg), protamine sulfate (50 mg), PLGA (500 mg) and a mixed solution of acetone and methanol (acetone: methanol (volume ratio) = 2:1, 50 mL) were mixed using a vortex mixer to obtain liquid F. The liquid F thus obtained was discharged to form droplets using a fine particle production device by a spray drying method, and dried to obtain fine particle G (yield: 29%). The conditions are as follows:

### - Fine particle preparation conditions -

Inlet temperature: 60°C
Outlet temperature: 50°C or less
Mean circulating air volume: 0.51 m³/min
Air pressure: 0.3 MPa
Nozzle diameter: 0.4 mm
Feed rate: 5 mL/min
Trap temperature: -2°C

### Test Example 4: Preparation of Compound 1-Containing Composition (Fine Particle H)

A compound 1 (100 mg), ε-polylysine (60 mg), PLGA (500 mg) and a mixed solution of acetone and methanol (acetone: methanol (volume ratio) = 2:1, 50 mL) were mixed using a vortex mixer to obtain liquid G. The liquid G thus obtained was discharged to form droplets using a fine particle production device by a spray drying method, and dried to obtain fine particle H (yield: 32%). The conditions are as follows:

### - Fine particle preparation conditions -

Inlet temperature: 60°C
Outlet temperature: 50°C or less
Mean circulating air volume: 0.51 m³/min
Air pressure: 0.3 MPa
Nozzle diameter: 0.4 mm
Feed rate: 5 mL/min
Trap temperature: -2°C

### Reference Example 3: Investigation of Compound 1-Containing Composition (Hydrogel a)

### Reference Example 3-1: Preparation of Compound 1-Containing Composition (Hydrogel a)

A compound 1 (1.5 mg), polyethylene oxide (13.5 mg) and purified water (0.9 mL) were mixed using a vortex mixer, and stored at 4°C overnight to obtain hydrogel a.

### Reference Example 3-2: Pharmacokinetic Evaluation of Compound 1-Containing Composition (Hydrogel a)

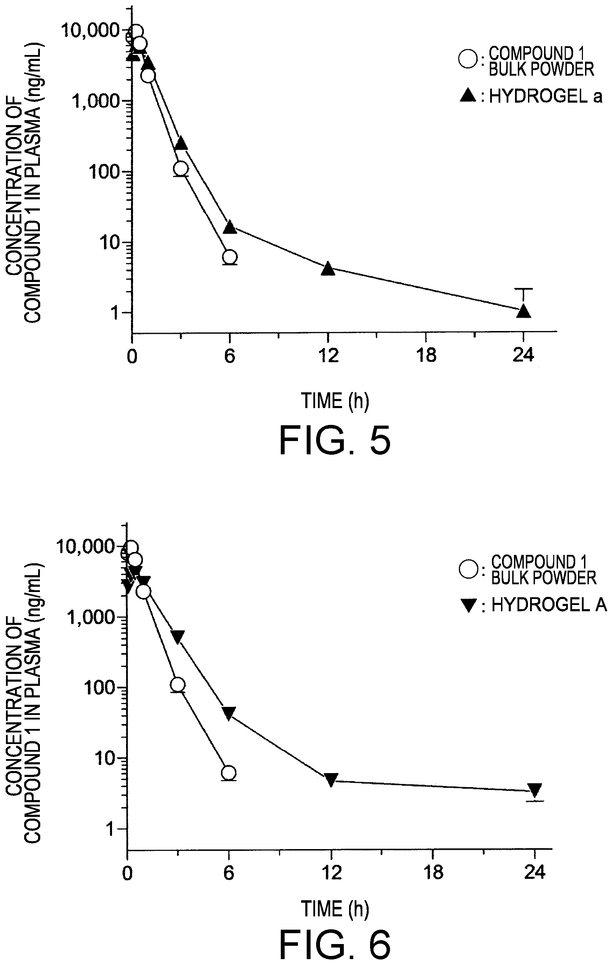

To evaluate the sustained-release ability of the hydrogel a prepared in Reference Example 3-1, the concentration of the compound 1 in blood was measured over time after subcutaneous administration of the hydrogel a or the compound 1 bulk powder to rats. Specifically, the hydrogel a or the compound 1 bulk powder at a dose of 5 mg/kg as an amount of the compound 1 was singly subcutaneously administered to SD male rats. After subcutaneous administration, blood was collected over time from the tail vein, and the blood was transferred into a micro test tube treated with heparin, and immediately cooled in ice. After cooling in ice, the blood was immediately centrifuged at 4°C and 10,000 g for 10 minutes. The concentration of the compound 1 in plasma thus obtained was quantitatively determined using ultra-high performance liquid chromatography with a single quadrupole mass spectrometer as a detector. The results are shown in FIG. 5. The following Table 6 shows the pharmacokinetic parameters calculated from the results of blood drug concentration measurement.

**[Table 6]**

| | Cₘₐₓ (µg/mL) | Tₘₐₓ (h) | T_{1/2} (h) |
|---|---|---|---|
| Compound 1 bulk powder | 9.6 ± 0.7 | 0.25 ± 0.0 | 0.36 ± 0.0 |
| Hydrogel a | 6.29 ± 0.4 | 0.31 ± 0.1 | 0.55 ± 0.0 |

In Table 6, pharmacokinetic parameter was expressed as mean ± standard error (n = 4 to 6).

From the results of the above Table 6 and FIG. 5, the hydrogel a prepared in Reference Example 3 had decreased absorption and elimination velocity of the compound 1, compared with the compound 1 bulk powder. Here, the Cₘₐₓ of the hydrogel a was decreased by 34.5% compared with that of the compound 1 bulk powder. The T_{1/2} of the hydrogel a was prolonged by 0.19 hour compared with that of the compound 1 bulk powder, and the hydrogel a of Reference Example 3 showed increased retention in blood of the compound 1. This is estimated to be due to the fact that the hydrogel a prepared in Reference Example 3 has a sustained-release drug elution property. It was confirmed that the hydrogel a has sustained release, but it was considered that there is room for improvement from a practical point of view.

### Test Example 5: Investigation of Compound 1-Containing Composition (Hydrogel A)

### Test Example 5-1: Preparation of Compound 1-Containing Composition (Hydrogel A)

A compound 1 (1.5 mg), polyethylene oxide (13.5 mg), ε-polylysine (1.5 mg) and purified water (0.9 mL) were mixed using a vortex mixer, and stored at 4°C overnight to obtain hydrogel A.

### Test Example 5-2: Pharmacokinetic Evaluation of Compound 1-Containing Composition (Hydrogel A)

To evaluate the sustained-release ability of the hydrogel A prepared in Test Example 5-1, the concentration of the compound 1 in blood was measured over time after subcutaneous administration of the hydrogel A or the compound 1 bulk powder to rats. Specifically, the hydrogel A or the compound 1 bulk powder at a dose of 5 mg/kg as an amount of the compound 1 was singly subcutaneously administered to SD male rats. After subcutaneous administration, blood was collected over time from the tail vein, and the blood was transferred into a micro test tube treated with heparin, and immediately cooled in ice. After cooling in ice, the blood was immediately centrifuged at 4°C and 10,000 g for 10 minutes. The concentration of the compound 1 in plasma thus obtained was quantitatively determined using ultra-high performance liquid chromatography with a single quadrupole mass spectrometer as a detector. The results are shown in FIG. 6. The following Table 7 shows the pharmacokinetic parameters calculated from the results of blood concentration measurement of the compound 1.

**[Table 7]**

| | Cₘₐₓ (µg/mL) | Tₘₐₓ (h) | T_{1/2} (h) |
|---|---|---|---|
| Compound 1 bulk powder | 9.6 ± 0.7 | 0.25 ± 0.0 | 0.36 ± 0.0 |
| Hydrogel A | 4.23 ± 0.4 | 0.38 ± 0.1 | 0.84 ± 0.1 |

In Table 7, pharmacokinetic parameter was expressed as mean ± standard error (n = 4 to 6).

From the results of the above Table 7 and FIG. 6, the hydrogel A prepared in Test Example 5 had decreased absorption and elimination velocity of the compound 1, compared with the compound 1 bulk powder. Here, the Cₘₐₓ of the hydrogel A was decreased by 56% compared with that of the compound 1 bulk powder. The T_{1/2} of the hydrogel A was prolonged by 0.48 hour compared with that of the compound 1 bulk powder, and the hydrogel A of Test Example 5 showed increased retention in blood of the compound 1. At 12 hours after administration, the concentration of the compound 1 in plasma in the compound 1 bulk powder was below the detection limit, while the concentration of the compound 1 in plasma in the hydrogel A was 5 ng/mL. This is estimated to be due to the fact that the hydrogel A prepared in Test Example 5 has a sustained-release drug elution property.

The Tₘₐₓ and the T_{1/2} of the hydrogel A in Test Example 5 were prolonged compared with the Tₘₐₓ and the T_{1/2} of the hydrogel a of Reference Example 3, respectively.

## Claims

1. A composition for topical administration, comprising
N-(2-ethylsulfonylamino-5-trifluoromethyl-3-pyridyl)cyclohe xanecarboxamide or a salt thereof, and
a polycation polymer.

2. The composition according to claim 1, wherein the polycation polymer is selected from the group consisting of a copolymer of an acrylic ester and a methacrylic ester having a positively charged nitrogen atom-containing group, polyamino acid, polyamine, polyamidoamine, polyimine, chitosan, poly N,N-dimethylaminoethyl methacrylate, polyvinylpyridine, polyimidazole, polyvinylamine, polyvinylformamide, protamine, polythiodiethylaminomethylethylene, poly-p-aminostyrene, polycation carbohydrate, polycation polymethacrylate, polycation polyacrylate, polycation polyoxetane, a derivative thereof and a salt thereof and a combination thereof.

3. The composition according to claim 2, wherein the copolymer of an acrylic ester and a methacrylic ester having a positively charged nitrogen atom-containing group is an ethyl acrylate-methyl methacrylate-trimethylammoniumethyl methacrylate copolymer.

4. The composition according to claim 2, wherein the polyamino acid is at least one selected from the group consisting of polylysine, polyarginine, polyhistidine and polyornithine.

5. The composition according to any one of claims 1 to 4, wherein the polycation polymer is a biodegradable polymer.

6. The composition according to any one of claims 1 to 5, wherein a form of the composition is a particle.

7. The composition according to claim 6, wherein the particle is in a form of being suspended in a solvent.

8. The composition according to any one of claims 1 to 5, wherein a form of the composition is a hydrogel.

9. The composition according to claim 8, wherein the hydrogel further comprises a hydrophilic polymer selected from the group consisting of poly(alkyleneoxide), poly(vinylalcohol), alginic acid, hyaluronic acid, chondroitin sulfate, gelatin, dextran, polyethylene glycol, methylcellulose, hydroxymethyl cellulose, hydroxyethyl cellulose, polyhydroxybutyrate, poly(n-isopropylacrylamide), carrageenan, pectin, dextran sulfate and a combination thereof.

10. The composition according to any one of claims 1 to 9, wherein the topical administration is subcutaneous administration, transrectal administration, intraperitoneal administration, intraarticular administration, intraocular administration, intratumoral administration, perivascular administration, intracranial administration, intramuscular administration, periocular administration, intrapalpebral administration, intraoral administration, intranasal administration, intravesical administration, intravaginal administration, intraurethral administration, intrarectal administration, adventitial administration or transnasal administration.

11. The composition according to any one of claims 1 to 10, which is a sustained-release composition.

12. The composition according to any one of claims 1 to 11, for treating or preventing a disease, a pathological condition or a symptom associated with an inflammatory cell.

13. The composition according to any one of claims 1 to 12, for a non-human animal.

14. A method for treating or preventing a disease, a pathological condition or a symptom associated with an inflammatory cell of a non-human animal, the method comprising topical administration of the composition according to any one of claims 1 to 13 to the non-human animal.

15. A method for producing the composition according to any one of claims 1 to 14, the method comprising
preparing a mixture comprising N-(2-ethylsulfonylamino-5-trifluoromethyl-3-pyridyl)cyclohexaneca rboxamide or a salt thereof and a polycation polymer.

16. The production method according to claim 15, further comprising drying the mixture.

17. The production method according to claim 16, wherein the drying is selected from the group consisting of spray drying, freeze drying and a combination thereof.

18. The production method according to claim 15, wherein the mixture is a good solvent solution in which
N-(2-ethylsulfonylamino-5-trifluoromethyl-3-pyridyl)cyclohexaneca rboxamide or a salt thereof and a polycation polymer are dissolved, the method comprising
mixing the good solvent solution with a poor solvent.
